# EUROPÄISCHE PATENTSCHRIFT

(11) **EP 0 254 090 B1**
(45) Veröffentlichungstag und Bekanntmachung des Hinweises auf die Patenterteilung: **12.05.1993**
(21) Anmeldenummer: 87109492.6
(22) Anmeldetag: 01.07.1987
(51) Int. Cl.: C12N 15/62, C12P 21/00, C12N 9/52

(54) **Verfahren zur gentechnologischenGewinnung von Proteinen unter Verwendung gramnegativer Wirtzellen**
Process for recovering proteins by means of gene technology using gram-negative host cells
Procédé pour obtenir des protéines par génie génétique en utilisant des cellules-hôtes gram-négatives

(30) Priorität: 02.07.1986 DE 3622221
(43) Veröffentlichungstag der Anmeldung: 27.01.1988
(73) Patentinhaber: Max-Planck-Gesellschaft zur Förderung der Wissenschaften e.V., D-37073 Göttingen (DE)
(72) Erfinder: Meyer, Thomas Ferdinand, Dr., D-7400 Tübingen (DE); Halter, Roman, D-7400 Tübingen (DE); Pohlner,Johannes, D-7400 Tübingen (DE)
(74) Vertreter: Huber, Bernhard, Dipl.-Chem.

(56) Entgegenhaltungen:
- PROCEEDINGS OF THE NATIONAL ACADEMY OF SCIENCES OF THE USA, Band 79, Dezember 1982, Seiten 7881-7885; J.M. KOOMEY et al.: "Genetic and biochemical analysis of gonococcal IgA1 protease: Cloning in EScherichia coli and construction of mutants of gonococci that fail to produce the activity"
- CHEMICAL ABSTRACTS, Band 101, Nr. 15, 8. Oktober 1984, Seite 155, Zusammenfassung Nr. 123972e, Columbus, Ohio, US; R. HALTER et al.: "IgA protease of Neisseria gonorrhoeae: isolation and characterization of the gene and its extracellular product", & EMBO J. 1984, 3(7), 1595-601
- NATURE, Band 325, 29. Januar 1987, Seiten 458-462; J. POHLNER et al.: "Gene structure and extracellular secretion of Neisseria gonorrhoeae IgA protease"

## Beschreibung

Die Erfindung betrifft ein Verfahren zur gentechnologischen Gewinnung von Proteinen unter Verwendung gramnegativer Wirtszellen, in welche ein rekombinanter Vektor eingeführt wird, der wenigstens ein das gewünschte Protein codierendes Gen enthält, Transkription dieses Gens und Translation.

Gentechnologische Verfahren zur Gewinnung von Proteinen sind seit langem bekannt. Vorzugsweise werden dazu Mikroorganismen verwendet, die leicht zu züchten sind und die die Gewinnung des erzeugten Proteins in einfacher Weise gestatten. Ein vielverwendeter Mikroorganismus ist dabei der E.coli, der sich sehr leicht züchten läßt und dessen Eigenschaften sehr gut bekannt sind. Nachteil dieser Mikroorganismengattung ist es, daß sie als gramnegative Mikroorganismen die erzeugten Proteine bzw. Polypeptide oft nicht in die Umgebung ausschleußen, sondern allenfalls in das Periplasma abgeben. Die Aufarbeitung ist daher mühsam und kann nur durch Zerstörung der Mikroorganismen erfolgen.

Es werden zwar eine Vielzahl von Proteine in gramnegativen Bakterien sezerniert. Es zeigte sich jedoch, daß für die Sekretion dabei spezifische bakterielle Proteine für den zweiten Translokationsschritt durch die äußere Membran notwendig sind. Die Klonierung der entsprechenden Strukturgene führte zu der Akkumulation dieser Proteine im Periplasma von E.coli. Derselbe Effekt wird auch für die Haemophilus influenzae IgA-Protease beschrieben, die im natürlichen Wirt extrazellulär sezerniert, aber wenn sie in E.coli kloniert wird, im Periplasma gefunden wird. Noch komplexer ist die Sekretion des E.coli-Haemolysins.

Hier sind insgesamt 4 verschiedene Proteinkomponenten, die auf einem einzigen Operon codiert sind, erforderlich.

Es war daher Aufgabe der vorliegenden Erfindung, ein Verfahren zur gentechnologischen Gewinnung von Proteinen unter Verwendung gramnegativer Wirtszellen so zu verbessern, daß das gewünschte Protein nach seiner Bildung in der Wirtszelle aus der Zelle ausgeschleußt wird und dann extrazellulär aus dem Kulturmedium gewonnen werden kann.

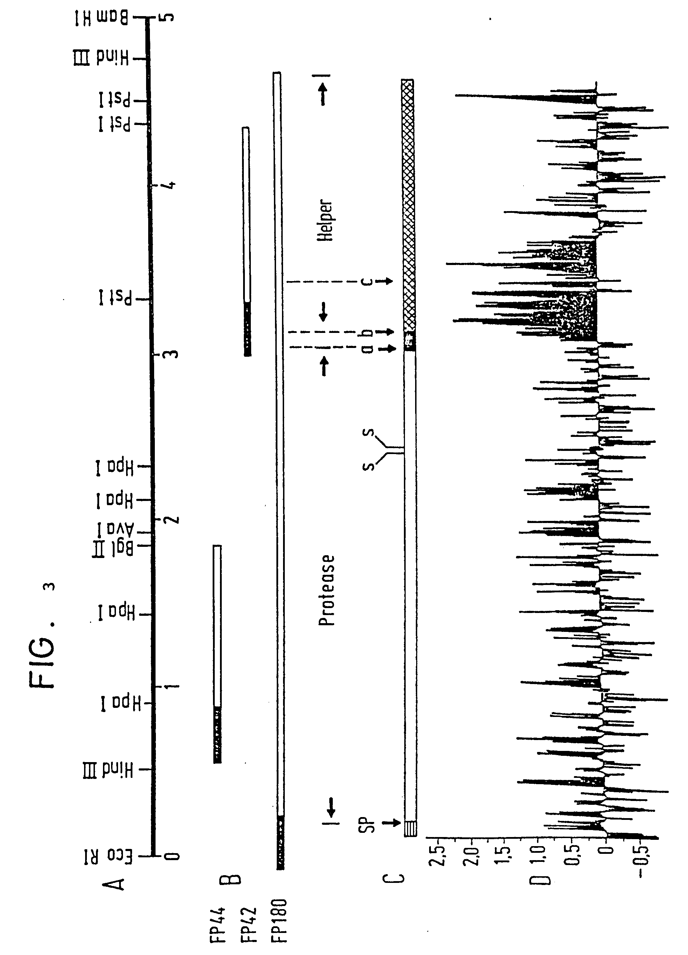

Diese Aufgabe wurde gelöst durch ein Verfahren zur gentechnologischen Gewinnung von Proteinen unter Verwendung gramnegativer Wirtszellen, in welche ein rekombinanter Vektor eingeführt wird, der wenigstens ein das gewünschte Protein codierendes Gen enthalt, Transkription dieses Gens und Translation, das dadurch gekennzeichnet ist, daß man zur extrazellulären Gewinnung des Proteins ein dafür codierendes Gen in einen Vektor derart einsetzt, daß eine rekombinante DNA entsteht, die für ein Fusionspolypeptid codiert, wobei dieses (i) einen N-terminalen Leaderanteil zum Durchdringen innerer Membranen der gramnegativen Wirtszellen, (ii) einen Anteil, der das gewünschte Protein enthält, und (iii) einen C-terminalen Anteil aus dem Helper-Segment eines IgA-Protease-Precursor-Gens gemäß Figur 3 zum Durchdringen äußerer Membranen der gramnegativen Wirtszellen enthält, wobei der Anteil aus dem Helper-Segment so gewählt ist, daß er eine extrazelluläre Sekretion des Fusionspolypeptids bewirkt.
Dabei kann, in einer bevorzugten Ausführungsform des Verfahrens ein N-terminaler Leaderanteil aus dem IgA-Protease-Precursor-Gen verwendet werden. Ebenso ist es jedoch möglich, daß man einen N-terminalen Leaderanteil verwendet, der nicht aus dem IgA-Protease-Precursor-Gen stammt.

Verschiedene pathogene Bakterienarten der Gattung Neisseria wie Neisseria gonorrhoeae und Neisseria meningitidis, die auf der menschlichen Schleimhaut wachsen, geben extrazellulär Proteasen ab, die spezifisch sind für menschliches IgA 1. Dieses Immunglobulin ist neben IgA 2 die Hauptkomponente der sekretorischen Immunität, die gegen Infektionen mit solchen Pathogenen schützen soll. Verwandte Bakterienarten, die nicht pathogen sind, produzieren keine IgA-Proteasen.

Das aus Neisseria gewonnene IgA-Protease-Gen Zeigt überraschende Eigenschaften. Die gebildete IgA-Protease bzw. ihr Precursor kann aktiv sezerniert werden, nicht nur in dem natürlichen Wirt, sondern auch in fremden, gramnegativen Wirtszellen, wie Enterobacteriaceae. Ein einziges Gen, das einen großen Precursor codiert, genügt für Produktion und extrazelluläre Sekretion von IgA-Protease auch in einer fremden Wirtszelle. Dieser Precursor, der mit einem Molekulargewicht von 170 kd annähernd 63 kd größer ist als die fertige extrazelluläre IgA-Protease, die nur ein Molekulargewicht von 106 kd hat, wird beim Transport durch die Membran der gramnegativen Wirtszelle und durch Prozessierung aufgrund der autoproteolytischen Aktivität des Enzyms in die fertige, aktive Form der IgA-Protease überführt unter Abspaltung einiger Teile des Precursormoleküls. Dieses Precursorprotein wird codiert von einem DNA-Fragment mit 5 Kb.

Der Precursor bildet 3 funktionelle Domänen, ein gleichmäßiges aminoterminales Leaderpeptid, den tatsächlichen Proteaseanteil und einen relativ großen carboxyterminalen Helperanteil.

Das Leaderpeptid führt den IgA-Proteaseprecursor in den Periplasmaspalt und wird von dem Precursor durch eine Signalpeptidase der inneren Membran entfernt. Der Initialschritt beim Transport der IgA-Protease könnte deshalb einem regulären Co- oder Posttranslationsweg gleichen, der durch die meisten der Periplasma- und Außenmembranproteine befolgt wird.

In einem zweiten Schritt wird der verbleibende Precursor-Komplex, der aus Protease- und Helper-Anteil besteht, in die äußere Membran transportiert und extrazellulär freigegeben. Der mechanistische Verlauf dieses Prozesses kann mit der Helperfunktion des IgA-Protease-Precursor-Komplexes erklärt werden. Der aminoterminale Helperanteil ist stark polar, wogegen der carboxyterminale Anteil eher hydrophob ist und Merkmale zeigt, die typisch sind für eine Membrananlagerung. Eine Erklärung, die aus diesen Beobachtungen resultiert, ist, daß der Helper eine Pore in der äußeren Membran bildet, wobei die hydrophilen Teile in diese Pore ragen. Der tatsächliche Proteaseanteil, der immer noch mit dem Helper verbunden ist, wird dann durch diese Pore geschoben und nimmt extrazellulär seine aktive Konformation an. Eine sequenzielle autoproteolytische Prozessierung entläßt dann die fertige, aktive Form der IgA-Protease in die extrazelluläre Umgebung. Die Energie, die für den aktiven Transport erforderlich ist, stammt von der autoproteolytischen Abspaltung des Helpers.

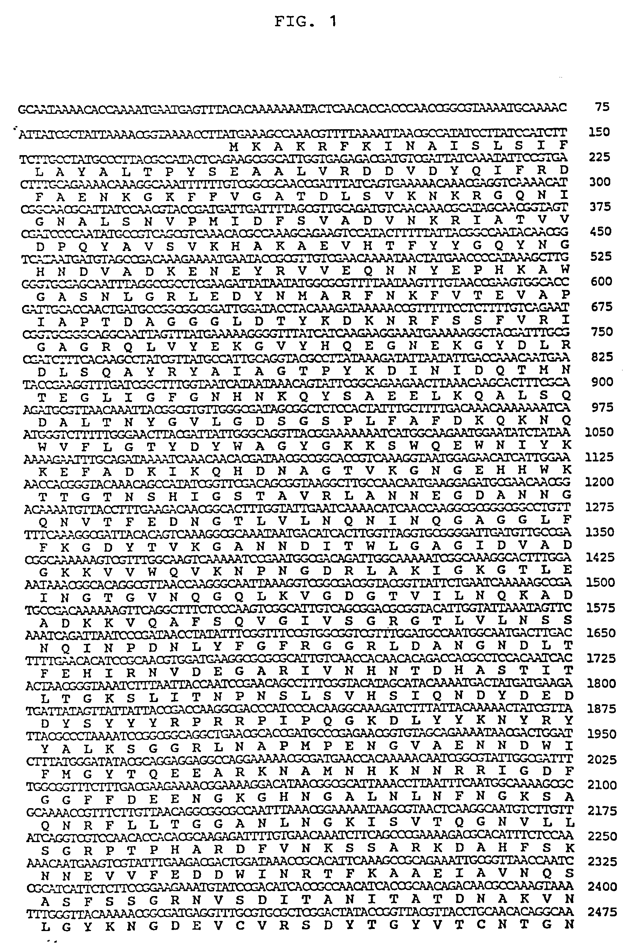

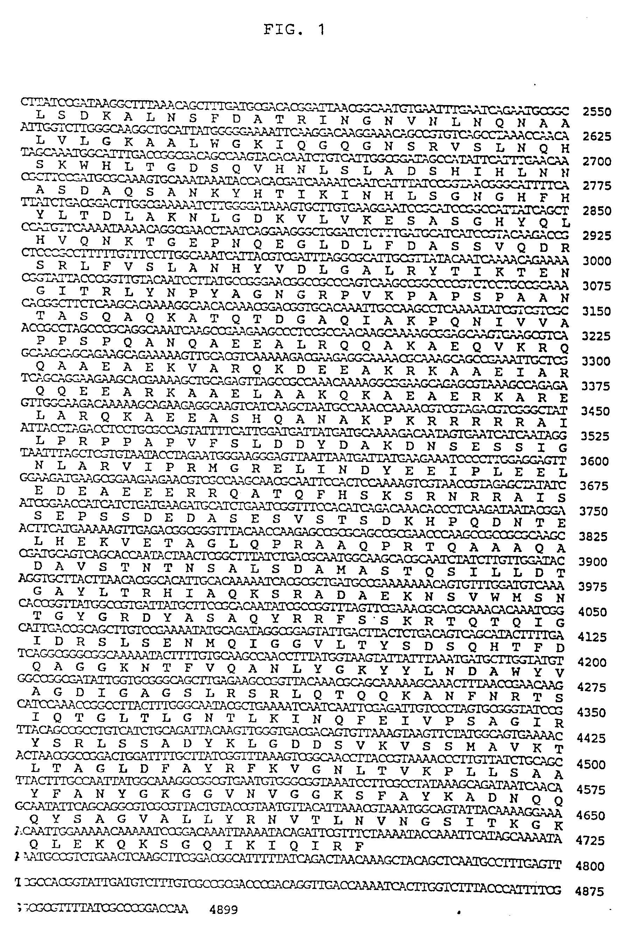

Zur extrazellulären Gewinnung eines gewünschten Proteins wird in an sich bekannter Weise dessen codierendes Gen in einen Vektor, der das IgA-Proteasegen der Neisseria enthält, eingesetzt. Die Sequenz dieses IgA-Proteasegens besteht, wie oben ausgeführt, aus 3 Hauptdomänen, dem Leaderanteil, der Sequenz, die für die IgA-Protease selbst codiert und dem Helperanteil. Es wurde nun überraschenderweise festgestellt, daß sich zwischen der die Protease codierenden Region und der Helperdomäne bzw. innerhalb der Helperdomäne drei natürliche Spaltstellen befinden. Diese Spaltstellen, die in der in Figur 1 dargestellten Gensequenz mit a, b und c bezeichnet sind, werden von den folgenden Aminosäuren gebildet:
Spaltstelle (a): Pro-Ala-Pro-Ser-Pro
Spaltstelle (b): Pro-Pro-Ser-Pro
Spaltstelle (c): Pro-Arg-Pro-Pro-Ala-Pro.

Die codierende Sequenz für das gewünschte Protein kann nun in verschiedene Bereiche des IgA-Proteasegens eingesetzt werden. Eine Insertion in den Leaderanteil kommt nicht inbetracht, da dieser Anteil während des Durchgangs durch die innere Membran abgespalten wird.

Eine Variante des erfindungsgemäßen Verfahrens besteht nun darin, das für das gewünschte Protein codierende Gen in die IgA-Protease-Domäne zu insertieren. An bestimmten Stellen der Protease-Domäne des IgA-Protease-Precursor-Gens ist dieser Einbau ohne Zerstörung der IgA-Proteaseaktivität möglich. In diesem Fall wird der IgA-Proteaseprecursor exprimiert, wobei sich das gewünschte Protein innerhalb des Proteaseanteils befindet.

Die noch inaktive IgA-Protease, bzw. deren Precursor wird ausgeschleust in der oben beschriebenen Art und Weise. Das gewünschte Protein liegt dann gebunden an die zumindest partiell noch aktive Protease vor. Die Trennung von der Protease erfolgt dann entweder durch Prozessierung der Protease, durch eine dafür geeignete Protease oder durch chemische Reaktionen.

Das für das gewünschte Protein codierende Gen kann auch so in die IgA-Protease-Domäne insertiert werden, daß keine aktive IgA-Protease mehr gebildet werden kann und somit IgA-Proteaseaktivität nicht mehr vorhanden ist. In die- sem Fall besteht eine bevorzugte Ausführungsform des erfindungsgemäßen Verfahrens darin, entweder in dasselbe Bakterium oder in ein anderes Bakterium, das gleichzeitig gezüchtet wird, einen Vektor, der das gesamte IgA-Proteasegen trägt, einzuführen. Die dann gleichzeitig mit dem gewünschten Protein gebildete IgA-Protease kann das gewünschte Protein von dem Helper und damit von der Membranwand abtrennen und damit die Gewinnung aus dem Kulturmedium ermöglichen.

In einer bevorzugten Ausführungsform des erfindungsgemäßen Verfahrens wird die Gensequenz, die das gewünschte Protein codiert, in einen Genabschnitt des IgA-Protease-Precursor-Gens eingefügt, der zwischen den natürlichen Spaltstellen (a), (b) und/oder (c) liegt. In diesem Falle bleibt die Aktivität der IgA-Protease erhalten und das gewünschte Protein wird nach Ausschleusung durch die beiden Membranen bei der Prozessierung des Precursors durch die natürliche Abspaltung auch von der Protease abgetrennt und liegt frei im Kulturmedium vor, aus dem sie dann in bekannter Weise isoliert werden kann.

Der Einbau in die Proteasedomäne des IgA-Gens kann auch so erfolgen, daß Teile oder die gesamte IgA-Proteasedomäne zerstört werden. Durch Zerstörung der IgA-Proteaseaktivität kommt es zwar zu einem Ausschleusen des gewünschten Proteins durch die beiden Membranen des gramnegativen Bakterienstammes, da jedoch die Proteaseaktivität fehlt, die den an der Membran assoziierten Helper von dem Enzym trennt, bleibt das gewünschte Protein an den Helper gebunden und damit an der Zellwand assoziiert. Diese Ausführungsform des erfindungsgemäßen Verfahrens ist besonders geeignet zur Herstellung von Lebendvakzine. Auf diese weise kann beispielsweise ein Oberflächenantigen an die äußere Membran des gramnegativen Mikroorganismus gebunden werden und zur Erzeugung von Antikörpern verwendet werden.

Zur erfindungsgemäßen Gewinnung der gewünschten Proteine sind gramnegative Wirtszellen, insbesondere Mikroorganismen der Familie Enterobacteriaceae sehr gut geeignet. Bevorzugt werden Mikroorganismen der Gattungen Escherichia und Salmonella eingesetzt. Eine Untersuchung mit den Stämmen E. coli, Salmonella typhimurium und abgeschwächten Salmonella typhii ergab, daß mit diesen Wirtszellen sogar höhere Aktivitäten an IgA-Protease in den Überstand abgegeben werden als bei N. gonorrhoeae, dem natürlichen Wirt. Die Sekretion der IgA-Protease in all diesen gramnegativen Systemen beruht auf demselben Mechanismus, da das extrazelluläre Enzym identisch ist und aus demselben Precursor prozessiert wird.

Das erfindungsgemäße Verfahren eignet sich zur Gewinnung von Proteinen. Besonders bevorzugt werden mit Hilfe des erfindungsgemäßen Verfahrens biologisch oder therapeutisch aktive Proteine gewonnen. Eine weitere Anwendung des erfindungsgemäßen Verfahrens besteht darin, antigene Epitope zur Anwendung in gramnegativen bakteriellen Lebendimpfstoffen auszuschleusen.

Das erfindungsgemäße Verfahren wird besonders bevorzugt durchgeführt mit E.coli DSM 3775 als Wirtszelle, der das Plasmid pIP 100 enthält. Das Plasmid pIP100 leitet sich ab von dem Plasmid pBR 322 und trägt die gesamte Gensequenz des IgA-Protease-Precursors (EMBO Journal, 1984, 3(7) S, 1595-1601).

Das erfindungsgemäße Verfahren ermöglicht es, in einfacher Weise Proteine zu gewinnen. Die Proteine können aus dem Kulturmedium gewonnen werden, obwohl als Wirtszellen gramnegative Bakterien verwendet werden, die leicht zu züchten sind.

Das erfindungsgemäß verwendete für den IgA-Protease-Precursor codierende DNA-Fragment hat eine Gesamtsequenz von 4899 Basenpaaren, die einen einzigen zusammenhängenden offenen Ableserahmen von 4602 Basenpaaren Länge bilden. Die Gensequenz ist der Figur 1 zu entnehmen.

Das ATG-Startkodon, das das dritte Kodon des offenen Leserahmens ist und eine Sequenz, die der Shine-Dalgarno-Konsensussequenz ähnlich ist, zeigt eine Translationsinitiationsstelle an Position 104. Die Translation endet mit einem TAA-ochre-Stopkodon bei Position 4700. Eine AT-reiche Region vor dem Translationsinitiationssignal zeigt typische Merkmale eines Promotors und dirigiert die Transkription des IgA-Proteasegens. Eine Palindromsequenz, die dem Translationsstopkodon folgt, kann als Transkriptionsterminator dienen.

Eine präzise Auswertung der Nukleotidsequenzdaten ergab eine Gesamtheit von 1532 Aminosäuren für den IgA-Proteaseprecursor, was ein Protein mit 169 kd ergibt (Figur 1). Die vollständige Precursorsequenz enthält nur zwei Cysteinreste, die in einem Abstand von 11 Aminosäuren gefunden wurden. Dies ist interessant, da die Abwesenheit von Cystein charakteristisch ist für viele sekretorische Proteine. Das Hydrophilieprofil ergibt ein sehr regelmäßiges abwechselndes Muster mit einigen herausragenden Merkmalen: ein kurzes hydrophobes Segment am aminoterminalen Ende bildet ein typisch sekretorisches Signalpeptid, das 4 positive aminoterminale Ladungen und eine zentrale hydrophobe Region zeigt. Die Spaltstelle dieses als Leaderpeptids bezeichneten Segmentes befindet sich zwischen 2 Alaninresten an den Positionen -1 und +1 (Figur 1). Die auffallendsten Merkmale der Primärstruktur des Precursors sind jedoch zwei hervorragende hydrophile Regionen rechts von der Zentralregion des Precursors und eine kurze an ihrem Carboxyende.

Der Teil, der der fertigen extrazellulären IgA-Protease entspricht, befindet sich im Anschluß an das Leaderpeptid im aminoterminalen Bereich des Precursormoleküls.

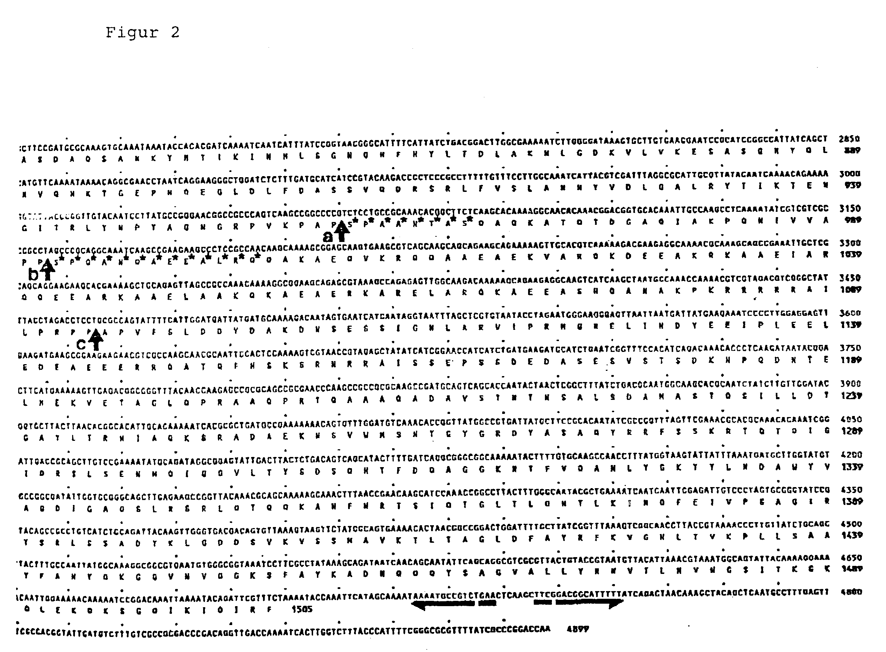

Die Grenzregion zwischen Protease und Helper enthält verschiedene prolinreiche Bereiche, die auffallende Sequenzübereinstimmungen mit der Spaltstelle der IgA-Protease aus N. gonorrhoeae bei humanem IgA 1 zeigen. Figur 3 zeigt die Aminosäuresequenzen dieser Stellen, die mit a, b und c bezeichnet werden (Figur 1). Diese Stellen sind die autoproteolytischen Angriffspunkte der IgA-Protease. Um dies zu bestätigen, wurde die Spaltung mit dem Enzym in Trans untersucht. Zu diesem Zweck wurden MS2/IgA-Proteasefusionsproteine, in präparativen Mengen auf Gel isoliert und mit geringen Mengen gereinigter IgA-Protease inkubiert. Die Inkubation verschiedener IgA-Proteasefusionen mit aktivem Enzym führte tatsächlich zu spezifischen Abbauprodukten. Das in Figur 2 gezeigte Fusionsprotein fp 180, wurde in zwei Hauptprodukte mit 120 kd und 60 kd und zwei kleinere Produkte von 45 kd und 15 kd gespalten. Kontrollinkubationen sind negativ, wie es für eine spezifische Reaktion mit IgA-Protease erwartet wird. Unter Verwendung eines monoklonalen Antikörpers, der gegen die fertige Protease gerichtet ist, reagiert die 120 kd-Bande im Immunoblot. Antiserum, das gegen das fp 42 Fusionsprotein (Figur 2) gerichtet ist, ergibt eine Kreuzreaktion mit den 60 kd und 45 kd Produkten und ebenso mit dem 15 kd Produkt. Die Kreuzreaktion mit dem 120 kd Produkt beruht auf dem MS2 Polymeraseanteil in den Fusionsproteinen. Diese Daten bestätigen, daß die Stellen (a) und/oder (b) tatsächlich die Angriffspunkte der IgA-Protease sind. Die 45 kd und 15 kd Produkte ergeben sich wahrscheinlich durch teilweise Verdauung an der Stelle (c) (Figur 2). Neben diesen Stellen werden offensichtlich keine anderen Regionen in den Fusionsproteinen als Angriffspunkte von der IgA-Protease akzeptiert.

Detailliertere Informationen über die internen Precursorangriffspunkte wurden durch eine aminoterminale Sequenzanalyse des 60 kd Spaltproduktes erhalten (Figur 3). Dieses Proteinfragment erschien als eine diffuse Bande in dem Acrylamidgel und nach der Genkarte stellt sie den carboxyterminalen Teil des Precursors dar (Figur 1). Die Analyse ergab unzweideutige Daten für die Sequenzierungsschritte 1, 2, 4, 5, 7 und 9 bis 13. Für alle anderen Schritte bis zur Position 8 ergab die Sequenzanalyse Signale von zwei Aminosäuren. Jeweils eine dieser beiden Aminosäuren an jeder Position fiel zusammen mit der Sequenz an der Stelle (a) bzw. der Stelle (b) (Figur 3). Die Positionen 9 bis 13 fallen zusammen mit der aminoterminalen Sequenz der Stelle (b). Die Heterogenität der Proteinsequenz bestätigt daher die Verwendung von zwei Stellen (a) und (b) als Angriffspunkte für die IgA-Protease.

Die Beobachtung, daß die fertige IgA-Protease, die aus Kulturüberständen von N. gonorrhoeae hergestellt wurde, ein heterogenes Protein ist, das eine 106/109 kd Doppelbande in Acrylamidgel bildet, kann erklärt werden durch heterogene Carboxytermini der fertigen IgA-Protease entsprechend entweder der Stelle (a) oder der Stelle (b). Das 3 kd Peptidsegment, das die Distanz zwischen den Stellen (a) und (b) überbrückt, dürfte verantwortlich sein für die Differenz in den Molekulargewichten der beiden Formen.

In den rohen-Kulturüberständen kann eine zusätzliche Bande von 121 kd gezeigt werden, die auch zur IgA-Protease gehören. Diese Bande kreuzreagiert nicht nur mit Antiproteaseserum, sondern auch mit Antiserum gegen das Fusionsprotein fp 42, was zeigt, daß das 121 kd Protein einen abgegrenzten Teil des Helpers trägt.

Gemäß seiner Größe befindet sich das Carboxyende des Zwischenprodukts exakt an der Position (c) (Figur 1), einem der internen Angriffspunkte des IgA-Proteaseprecursors.

Es wurden die relativen Mengen der 3 Formen der extrazellulären IgA-Protease in einer wachsenden E.coli-Kultur bestimmt. Die Ergebnisse dieses Experimentes zeigen, daß in der frühen Wachstumsphase das 121 kd Zwischenprodukt überwiegt. Wenn sich das Enzym ansammelt während der Zeit des Wachstums, überwiegt das 109 kd Zwischenprodukt und schließlich kann die fertige 106 kd Form von IgA-Protease klar nachgewiesen werden. Dies deutet auf eine langsame Umwandlung des hochmolekulargewichtigen Zwischenproduktes in das fertige Enzym. Damit stimmt die Beobachtung überein, daß das Zwischenprodukt mit 109 kd Molekulargewicht, das noch als Hauptfraktion in einigen IgA-Proteasepräparationen enthalten ist, langsam in die niedrigmolekulargewichtige Form ungewandelt wird nach längerer Lagerung.

Diese Experimente zeigen, daß zumindest der polare Teil des Helpers extrazellulär sezerniert wird zusammen mit der IgA-Protease selbst. Durch Immunoblotting konnte gezeigt werden, daß der kleine 12 kd Anteil des Helpers, der sich zwischen den internen Spaltstellen (b) und (c) befindet, tatsächlich in die extrazelluläre Umgebung zusammen mit der IgA-Protease freigegeben wird. Es wird geschlossen, daß die polaren Eigenschaften dieser Region besonders wichtig für die extrazelluläre Sekretion der IgA-Protease sind.

Es wird angenommen, daß die Pathogenität der Mikroorganismen der Gattung Neisseriea eventuell auf die Bildung der IgA-Protease zurückzuführen ist. Eine Möglichkeit zur Bekämpfung der von diesen Mikroorganismen verursachten, schweren Krankheiten besteht daher darin, die Spaltstellen des IgA-Protease-Precursors zu blockieren, so daß der Precursor nicht mehr gespalten werden kann, d.h. seine eigenen Spaltstellen nicht mehr erkennt und damit das aktive Enzym, das für die schädlichen Wirkungen verantwortlich gemacht wird, nicht entstehen kann.

Bevorzugt werden zur Blockierung der Spaltstellen des Precursors spezifisch mit den die Spaltstellen (a), (b) und/oder (c) bildenden Aminosäuresequenzen bindefähige Substanzen, insbesondere gegen diese Aminosäuresequenzen gerichtete Antikörper, verwendet.

Eine weitere Möglichkeit besteht darin, die Schnittstellen der IgA-Protease durch Zugabe von Peptidanaloga zu blockieren, die eine im Hinblick auf die Sequenz der Schnittstelle leicht modifizierte Aminosäuresequenz haben, so daß sie sich zwar an die Schnittstellen anlagern, aber nicht geschnitten werden können und somit an der Schnittstelle haften bleiben.

Die Erfindung wird durch die folgenden Beispiele in Verbindung mit der Zeichnung erläutert.

In der Zeichnung stellen dar:
Figur 1 zeigt die Nukleotid- und Aminosäuresequenzen der IgA-Protease von N. gonorrhoeae Stamm MS11. Die Aminosäuresequenz, die von dem offenen Leserahmen abgeleitet wurde, umfaßt einen Precursor der IgA-Protease. Die Nukleotidsequenz resultiert aus der Summe der Daten, die sich aus der Analyse überlappender Segmente des klonierten DNA-Fragments in pIP100 ergab. Zur Anwendung kamen hierbei Techniken nach Maxam und Gilbert, sowie nach Sanger et al.

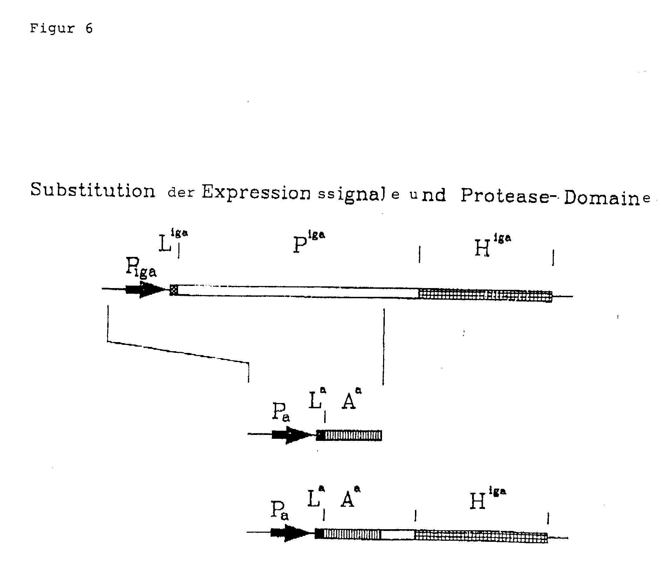

Figur 2 zeigt besondere Eigenschaften der Nukleotid- und Aminosäuresequenzen. Die Signale für den Start der Transkription (-43,-35 und -10) und Translation (SD) des IgA-Proteasegens sind durch dicke Striche gekennzeichnet. Ein Palindrom, das an der Termination der Transkription beteiligt ist, ist mit dicken Pfeilen unterlegt. Die aminoterminale Signalsequenz der IgA-Protease ist eingerahmt. Das Aminoende der reifen IgA-Protease wurde durch Proteinsequenzierung analysiert. Die Stellen (a), (b) und (c) zeigen die internen Spaltstellen der IgA-Protease (siehe auch Fig. 3 und 6). Die gespaltenen Peptidbindungen (gekennzeichnet durch Pfeile) wurden durch Proteinsequenzierung bestimmt und die Übereinstimmung dieser Daten mit der abgeleiteten Aminosäuresequenz ist durch Sternchen gekennzeichnet. Die zwei einzigen Cysteinreste des ganzen Precursors sind eingerahmt.
Figur 3 zeigt die physikalischen Eigenschaften der IgA-Protease und ihres Gens. A, Restriktionskarte des pIP100 Einschubs, das die Synthese und die Sekretion von Gonococcen IgA-Protease in E.coli bewirkt. B, Fusionsproteine des IgA-Proteaseprecursors hergestellt mit dem pEX Expressionssystem. Die dick ausgezogenen Linien zeigen den aminoterminalen MS 2 Polymerasecarrier des Expressionssystems, die offene Linie bezieht sich auf die entsprechenden Teile des Precursors, der in den Fusionsproteinen enthalten ist. Die Grenze zwischen Carrier und Precursoranteil entspricht der Stelle in dem IgA-Proteasegen, an der der Expressionsvektor angekoppelt wurde. C, lineare Karte des Precursors. Die gestrichelte Sektion bezieht sich auf das Leaderpeptid, die offene Sektion auf die Proteasedomäne und der gekreuzte Anteil auf die Helperdomäne. Pfeile zeigen die internen IgA-Proteaseschnittstellen (a, b und c). (SH) bezieht sich auf die Position der zwei einzigen Cysteine in dem Precursor und (x) zeigt die Stelle eines hypothetischen Translokationssignals der äußeren Membran. D, Hydropathyblot der Primärstruktur des Precursors.

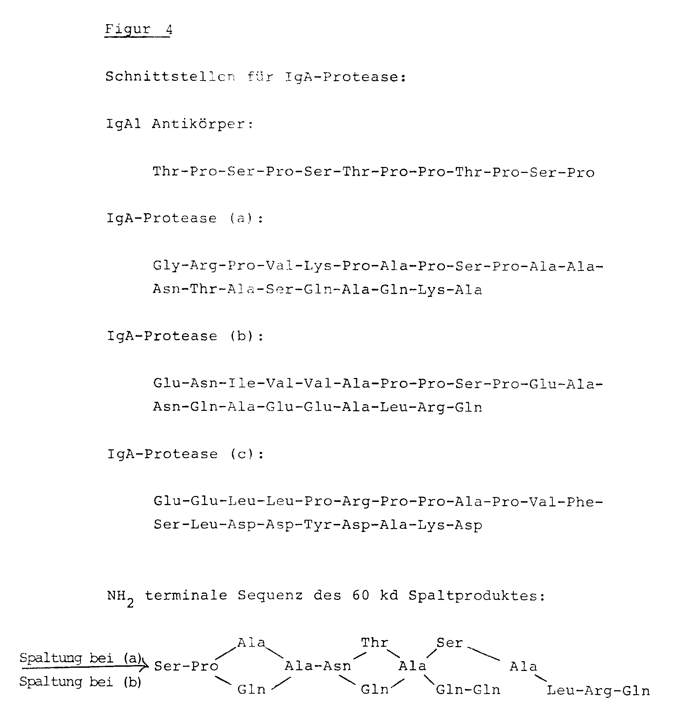

Figur 4 zeigt die Angriffspunkte für IgA-Protease und Proteinsequenzanalyse. A, Aminosäuresequenz der IgA-Proteasespaltstelle in menschlichem IgA 1 und Sequenz der Angriffspunkte in dem IgA-Proteaseprecursor (vergleiche Figuren 1 und 2). B, aminoterminale Sequenzanalyse des 60 kd Produktes, das bei der Spaltung des Fusionsproteins fp 180 mit gereinigter IgA-Protease entsteht. Quantitative Auswertung der Sequenzierungsdaten ergeben, daß 80 % des Produktes aus der Spaltung an der Schnittstelle (b) und nur 20 % von der Spaltung an der Stelle (a) resultieren.

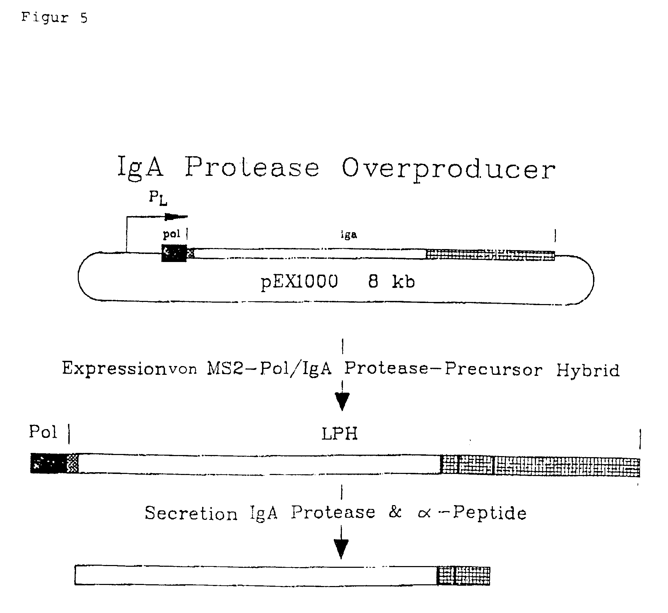

Figur 5 zeigt die Herstellung eines Vektors zur Überproduktion von IgA-Protease. Hierzu wurde das vollständige Gen des IgA-Proteaseprecursors unter die Kontrolle des pEX-Expressionssystems gestellt. Das Plasmid pEX1000 führt zur intrazellulären Bildung eines MS2-Polymerase/IGA-Proteaseprecursor-Hybridproteins. Im Verlauf des extrazellulären Transports kommt es zur Spaltung dieses Fusionsproteins und der Freigabe der IgA-Protease und desα-Proteins in den Kulturüberstand.
Figur 6 zeigt den Austausch der Expressionssignale sowie der IgA-Proteasedomäne in einem System zur Sekretion von Protein aus gramnegativen Bakterien. L^{iga}, P^{iga} und H^{iga} bezeichnen die Domänen des IgA-Proteaseprecursors. P_{iga} kennzeichnet den ursprünglichen Promoter des IgA-Proteasegens, der im konstruierten Hybridprotein gegen den Promoter (Pₐ) des Antigens A ausgetauscht wurde. L^{a} bezeichnet das Leaderpeptid des Antigens A, das für den Transport durch die innere Membran benötigt wird.

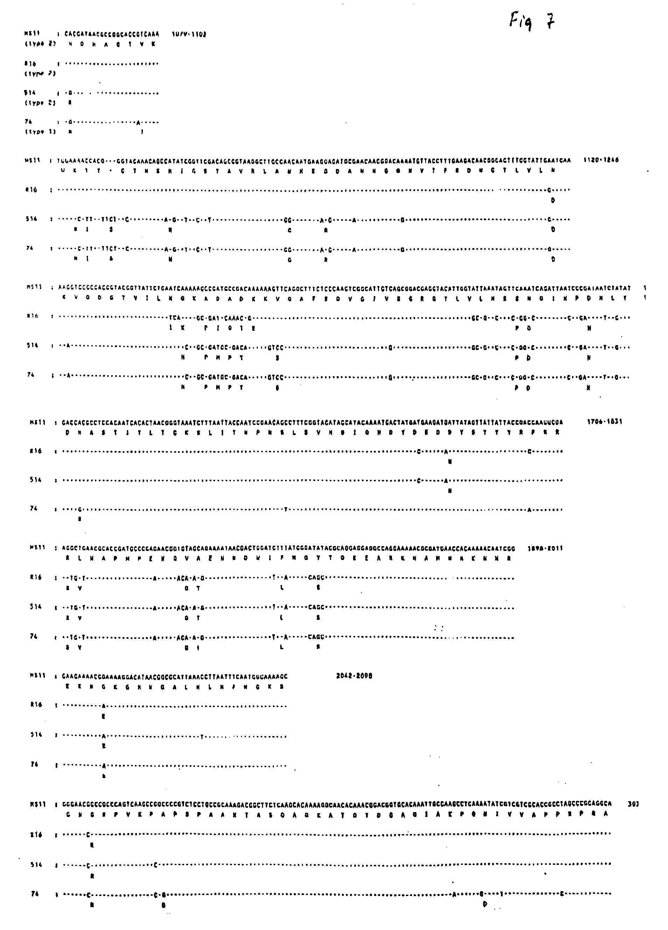

Figur 7 vergleicht teilweise sequenzierte IgA-Proteasegenabschnitte der Gonokokkenstämme R16 (Typ 1), 514 (Typ 2) und 74 (Typ 1) mit den entsprechenden Genabschnitten im IgA-Proteasegen des Gonokokkenstammes MS11. Die verglichenen Sequenzabschnitte umfassen die Nukleotidpositionen 1079-2140, 2410-2571 und 3011-3172. In der Abbildung sind davon jene Bereiche dargestellt, die auch Substitutionen von Nukleotiden aufweisen. Die Nukleotidpositionen sind in Figur 1 festgelegt. Insgesamt wurden zwischen 3 % bis 6 % Sequenzunterschiede zwischen den einzelnen Proteasegenen in den sequenzierten Genabschnitten nachgewiesen.

### Beispiel 1

### Überproduktion von IgA-Protease und Optimierung des Transports in den Kulturüberstand.

Mit der Hilfe synthetischer Oligonukleotide wurde das vollständige Gen des IgA-Protease-Vorläufers mit dem MS2-Polymerasegen im pEX-Expressionssystem fusioniert (siehe Figur 5). Das resultierende Plasmid pEX1000 führt im Cytoplasma der Wirtszellen zur Expression eines großen Hybridproteins bestehend aus den aminoterminalen 99 Aminosäuren der MS2-Polymerase, verbunden mit der gesamten Aminosäurefolge des IgA-Protease-Vorläufers. Dieses Fusionsprotein wird im Verlaufe der Sekretion proteolytisch gespalten und führt zur Freigabe der IgA-Protease sowie der Intermediate in den Kulturüberstand. Durch Deletion weniger Aminosäuren im sekretierten Segment des Helfers konnte sowohl die Effizienz als auch die Qualität der IgA-Proteasesekretion erheblich gesteigert werden (siehe Tabelle 1).

Tabelle 1 vergleicht die Iga-Protease-Sekretion verschiedener Bakterienarten sowie die-Auswirkungen von Änderungen der Expressionssignale sowie von Modifikationen im Gen der IgA-Protease. Die Proteinmengen wurden in SDS-Polyacrylamidgelen und Westernblot-Analysen bestimmt.

**Tabelle 1:**

| | eingefügte Aminosäuren | entfernte Aminosäuren | Wirksamkeit der Sekretion |
|---|---|---|---|
| N.gonorrhoeae MS11 | | | 0.05 mg/l |
| pIP100 (E.coli) | - | - | 0.05 |
| pIP100 (S.typhi. Ty21a) | - | - | 2.0 |
| pEX1000 | - | - | 0.5 |
| pEX1000.A51 | Arg-Asn-Ser-Gly⁽⁷⁷²⁾ | - | 0.5 |
| pEX1000.R9 | Ser-Gly-Ile-Pro⁽⁹⁴⁵⁾ | - | 0.5 |
| pEX1000.A36 | Ala-Gly-Ile-Pro⁽¹⁰⁷⁷⁾ | - | 0.2 |
| pEX1090 | - | 1080,1081 | 0.5 |
| pEX1080 | - | 1080-1083 | 0.7 |
| pEX1070 | - | 1080-1084 | 2.0 |
| pEX1072 | - | 1085-1084 | 2.0 |
| pEX1073 | - | 1048-1084 | 2.0 |

### Beispiel 2

### Einbau von Oligopeptiden in den IgA-Protease-Vorläufer und Transport in den Kulturüberstand.

Unter Verwendung gentechnologischer Methoden wurde in das Gen der IgA-Protease an drei Stellen ein synthetischer DNA-Doppelstrang der Länge 12 bp eingebaut. Dies entspricht nach Translation des Proteins einer Insertion von je vier zusätzlichen Aminosäuren. Alle drei Insertionen (siehe Tabelle 1) zeigen keinen Einfluß auf den Transport und die enzymatische Aktivität der IgA-Protease. Für die Plasmide pEX1000.A51, -.R9 und -.A36 (siehe Figur 5 und Tabelle 1) wurde in SDS-Polyacrylamidgelen und mit der Immunoblott-Technik gezeigt, daß im Überstand der Bakterien die gleichen Intermediate und der gleiche Endzustand der IgA-Protease auftreten wie im ursprünglichen Klon pIP100. Das insertierte Oligopeptid in pEX1000.A36 befindet sich zwischen den Schnittstellen (b) und (c) und wird somit nach Autoproteolyse in Verbund mit dem 12 kd Spaltprodukt löslich ins Medium abgegeben.

### Beispiel 3

### Substitution von Expressions-Signalen und der Protease-Domäne

Die carboxyterminale, 60 kd große Helfer-Domäne des IgA-Protease-Vorläuferproteins ist für den Transport der Protease-Domäne über die äußere Membran notwendig. Um die fundamentale Bedeutung des Helfers bei der Proteinsekretion zu bekräftigen, wurden mit gentechnologischen Methoden große Teile der Protease-Domäne P^{iga}, den Promotor P_{iga} und die Signalsequenz L^{iga} substituiert. Der Austausch erfolgte gegen ein Fragment, welches seinerseits die Signale für (i) Genexpression, Pₐ für (ii) den Proteintransport über die innere bakterielle Membran, die Signalsequenz L^{a} enthielt. Das Fragment lieferte zudem eine Sequenz (iii), die für einAntigen A^{a} kodierte. Translationale Fusionen mit der Helfersequenz H^{iga} erlaubten es, eine Helfer-spezifische Proteinbande von ungefähr 36 kd in Kulturüberständen rekombinanter E.coli-Zellen nachzuweisen. Dazu wurde ein monoklonaler Antikörper verwendet. Bisher gelang es nicht, ein vollständiges Fusionsprotein der erwarteten Größe nachzuweisen. Allerdings gelang der Nachweis eines 45 kd Helfer-spezifischen Proteins in Ganz-Zell-Lysaten rekombinanter Klone, nicht jedoch in deren Kulturüberständen. Aus dieser Beobachtung wird geschlossen, daß das 36 kd große, Helfer-spezifische Protein ein Degradationsprodukt der 45 kd Helferbande ist. Die beschriebene Degradation wurde bisher nur in rekombinanten E.coli-Zellen beobachtet, nicht jedoch im natürlichen Wirt.

## Patentansprüche (Patentansprüche für folgende(n) Vertragsstaat(en): BE, CH, DE, FR, GB, IT, LI, LU, NL, SE)

1. Verfahren zur gentechnologischen Gewinnung und extrazellulären Sekretion von Proteinen unter Verwendung gramnegativer Wirtszellen, in welche ein rekombinanter Vektor eingeführt wird, der wenigstens ein das gewünschte Protein kodierendes Gen enthält, Transkription dieses Gens und Translation,
**dadurch gekennzeichnet,**
daß man zur extrazellulären Gewinnung des gewünschten Proteins ein dafür kodierendes Gen in einen Vektor derart einsetzt, daß eine rekombinante DNA entsteht, die für ein Fusionspolypeptid kodiert, enthaltend:
(i) einen N-terminalen Leaderanteil zum Durchdringen innerer Membranen der gramnegativen Wirtszellen,
(ii) einen Anteil, der das gewünschte Protein enthält, und
(iii) einen C-terminalen Anteil aus dem Helper-Segment eines IgA-Protease-Precursor-Gens gemäß Figur 3 zum Durchdringen äußerer Membranen der gramnegativen Wirtszellen, wobei der Anteil aus dem Helper-Segment so gewählt ist, daß er eine extrazelluläre Sekretion des Fusionspolypeptids bewirkt.

2. Verfahren nach Anspruch 1,
**dadurch gekennzeichnet,**
daß man einen N-terminalen Leaderanteil aus dem IgA-Protease-Precursor-Gen verwendet.

3. Verfahren nach Anspruch 1,
**dadurch gekennzeichnet,**
daß man einen N-terminalen Leaderanteil verwendet, der nicht aus dem IgA-Protease-Precursor-Gen stammt.

4. Verfahren nach Anspruch 1,
**dadurch gekennzeichnet,**
daß man das für das gewünschte Protein kodierende Gen in einen Vektor, der das IgA-Protease-Precursor-Gen aus Mikroorganismen der Gattung Neisseria enthält, derart einsetzt, daß das kodierende Gen innerhalb der Sequenz für das Protease- oder/und das Helpersegment, aber nicht innerhalb der Sequenz für das Leadersegment des IgA-Protease-Precursor-Gens positioniert wird.

5. Verfahren nach einem der vorhergehenden Ansprüche,
**dadurch gekennzeichnet,**
daß man das für das gewünschte Protein kodierende Gen so einfügt, daß die entstehende rekombinante DNA ein für eine aktive IgA-Protease kodierendes Gensegment enthält.

6. Verfahren nach Anspruch 5,
**dadurch gekennzeichnet,**
daß man das kodierende Gen an einer Stelle des IgA-Protease-Precursor-Gens einfügt, die zwischen den sich in der Grenzregion von Protease- und Helpersegment befindlichen natürlichen Spaltstellen des IgA-Protease-Precursor-Proteins liegt.

7. Verfahren nach Anspruch 4,
**dadurch gekennzeichnet,**
daß man das kodierende Gen in das für die IgA-Protease kodierende Gensegment einführt.

8. Verfahren nach Anspruch 7,
**dadurch gekennzeichent,**
daß man zusätzlich in dieselbe Wirtszelle oder in eine weitere Wirtszelle, die gleichzeitig gezüchtet wird, einen Vektor einführt, der die IgA-Protease kodiert.

9. Verfahren nach Anspruch 7,
**dadurch gekennzeichnet,**
daß man die Freisetzung des an der Membran haftenden Proteins durch Zugabe einer spezifischen Protease, insbesondere IgA-Protease oder durch chemische Mittel bewirkt.

10. Verfahren nach einem der vorhergehenden Ansprüche,
**dadurch gekennzeichnet,**
daß als Wirtszelle ein Mikroorganismus der Familie Enterobacteriaceae verwendet wird.

11. Verwendung des Plasmids pIP100, enthalten in E.coli, DSM 3775 als Vektor für ein Verfahren nach einem der Ansprüche 1 bis 10.

12. EcoRI/BamHI-DNS-Fragment, das für einen IgA-Protease-Precursor aus Mikroorganismen der Gattung Neisseria kodiert, gemäß Fig. 3, aus pIP 100 (DSM 3775), enthaltend
a) die in Figur 1 gezeigte kodierende Nukleinsäuresequenz, oder
b) eine in Figur 7 gezeigte Variation dieser Sequenz

## Patentansprüche (Patentansprüche für folgende(n) Vertragsstaat(en): GR, AT, ES)

1. Verfahren zur gentechnologischen Gewinnung und extrazellulären Sekretion von Proteinen unter Verwendung gramnegativer Wirtszellen, in welche ein rekombinanter Vektor eingeführt wird, der wenigstens ein das gewünschte Protein kodierendes Gen enthält, Transkription dieses Gens und Translation,
**dadurch gekennzeichnet,**
daß man zur extrazellulären Gewinnung des gewünschten Proteins ein dafür kodierendes Gen in einen Vektor derart einsetzt, daß eine rekombinante DNA entsteht, die für ein Fusionspolypeptid kodiert, enthaltend:
(i) einen N-terminalen Leaderanteil zum Durchdringen innerer Membranen der gramnegativen Wirtszellen,
(ii) einen Anteil, der das gewünschte Protein enthält, und
(iii) einen C-terminalen Anteil aus dem Helper-Segment eines IgA-Protease-Precursor-Gens gemäß Figur 3 zum Durchdringen äußerer Membranen der gramnegativen Wirtszellen, wobei der Anteil aus dem Helper-Segment so gewählt ist, daß er eine extrazelluläre Sekretion des Fusionspolypeptids bewirkt.

2. Verfahren nach Anspruch 1,
**dadurch gekennzeichnet,**
daß man einen N-terminalen Leaderanteil aus dem IgA-Protease-Precursor-Gen verwendet.

3. Verfahren nach Anspruch 1,
**dadurch gekennzeichnet,**
daß man einen N-terminalen Leaderanteil verwendet, der nicht aus dem IgA-Protease-Precursor-Gen stammt.

4. Verfahren nach Anspruch 1,
**dadurch gekennzeichnet,**
daß man das für das gewünschte Protein kodierende Gen in einen Vektor, der das IgA-Protease-Precursor-Gen aus Mikroorganismen der Gattung Neisseria enthält, derart einsetzt, daß das kodierende Gen innerhalb der Sequenz für das Protease- oder/und das Helpersegment, aber nicht innerhalb der Sequenz für das Leadersegment des IgA-Protease-Precursor-Gens positioniert wird.

5. Verfahren nach einem der vorhergehenden Ansprüche,
**dadurch gekennzeichnet,**
daß man das für das gewünschte Protein kodierende Gen so einfügt, daß die entstehende rekombinante DNA ein für eine aktive IgA-Protease kodierendes Gensegment enthält.

6. Verfahren nach Anspruch 5,
**dadurch gekennzeichnet,**
daß man das kodierende Gen an einer Stelle des IgA-Protease-Precursor-Gens einfügt, die zwischen den sich in der Grenzregion von Protease- und Helpersegment befindlichen natürlichen Spaltstellen des IgA-Protease-Precursor-Proteins liegt.

7. Verfahren nach Anspruch 4,
**dadurch gekennzeichnet,**
daß man das kodierende Gen in das für die IgA-Protease kodierende Gensegment einführt.

8. Verfahren nach Anspruch 7,
**dadurch gekennzeichent,**
daß man zusätzlich in dieselbe Wirtszelle oder in eine weitere Wirtszelle, die gleichzeitig gezüchtet wird, einen Vektor einführt, der die IgA-Protease kodiert.

9. Verfahren nach Anspruch 7,
**dadurch gekennzeichnet,**
daß man die Freisetzung des an der Membran haftenden Proteins durch Zugabe einer spezifischen Protease, insbesondere IgA-Protease oder durch chemische Mittel bewirkt.

10. Verfahren nach einem der vorhergehenden Ansprüche,
**dadurch gekennzeichnet,**
daß als Wirtszelle ein Mikroorganismus der Familie Enterobacteriaceae verwendet wird.

11. Verwendung des Plasmids pIP100, enthalten in E.coli, DSM 3775 als Vektor für ein Verfahren nach einem der Ansprüche 1 bis 10.

## Claims (Claims for the following Contracting State(s): BE, CH, DE, FR, GB, IT, LI, LU, NL, SE)

1. Process for the gene-technological production and extracellular secretion of proteins with the use of grain-negative host cells, in which a recombinant vector is introduced which contains at least one gene coding the desired protein, transcription of this gene and translation, characterised in that, for the extracellular production of the desired protein, one uses a gene coding therefor in a vector in such a manner that a recombinant DNA results which codes for a fusion polypeptide, containing
(i) an N-terminal leader part for the penetration of inner membranes of the gram-negative host cells,
(ii) a part which contains the desired protein and
(iii) a C-terminal part from the helper segment of an IgA protease precursor gene according to Figure 3 for the penetration of outer membranes of the gram-negative host cells, whereby the part from the helper segment is so chosen that it brings about an extracellular secretion of the fusion polypeptide.

2. Process according to claim 1, characterised in that one uses an N-terminal leader part from the IgA protease precursor gene.

3. Process according to claim 1, characterised in that one uses an N-terminal leader part which does not originate from the IgA protease precursor gene.

4. Process according to claim 1, characterised in that one uses the gene coding for the desired protein in a vector which contains the IgA protease precursor gene from micro-organisms of the genus Neisseria in such a manner that the coding gene is positioned within the sequence for the protease and/or the helper segment but not within the sequence for the leader segment of the IgA protease precursor gene.

5. Process according to one of the preceding claims, characterised in that one so introduces the gene coding for the desired protein that the resultant recombinant DNA contains a gene segment coding for an active IgA protease.

6. Process according to claim 5, characterised in that one introduces the coding gene at a position of the IgA protease precursor gene which lies between the natural cleavage positions of the IgA protease precursor protein present in the boundary region of protease and helper segment.

7. Process according to claim 4, characterised in that one introduces the coding gene into the gene segment coding for the IgA protease.

8. Process according to claim 7, characterised in that one additionally introduces a vector which codes the IgA protease into the same host cell or into a further host cell which is cultured simultaneously.

9. Process according to claim 7, characterised in that one brings about the liberation of the protein adhering to the membrane by addition of a specific protease, especially IgA protease, or by chemical agents.

10. Process according to one of the preceding claims, characterised in that, as host cell, there is used a micro-organism of the family Enterobacteriaceae.

11. Use of the plasmid pIP100, contained in E. coli, DSM 3775, as vector for a process according to one of claims 1 to 10.

12. EcoRI/BamHI DNA fragment which codes for an IgA protease from micro-organisms of the genus Neisseria according to Fig. 3 from pIP 100 (DSM 3775), containing
a) the coding nucleic acid sequence shown in Figure 1 or
b) a variation of this sequence shown in Figure 7.

## Claims (Claims for the following Contracting State(s): GR, AT, ES)

1. Process for the gene-technological production and extracellular secretion of proteins with the use of gram-negative host cells, in which a recombinant vector is i roduced which contains at least one gene coding the desired protein, transcription of this gene and translation, characterised in that, for the extracellular production of the desired protein, one uses a gene coding therefor in a vector in such a manner that a recombinant DNA results which codes for a fusion polypeptide, containing
(i) an N-terminal leader part for the penetration of inner membranes of the gram-negative host cells,
(ii) a part which contains the desired protein and
(iii) a C-terminal part from the helper segment of an IgA protease precursor gene according to Figure 3 for the penetration of outer membranes of the gram-negative host cells, whereby the part from the helper segment is so chosen that it brings about an extracellular secretion of the fusion polypeptide.

2. Process according to claim 1, characterised in that one uses an N-terminal leader part from the IgA protease precursor gene.

3. Process according to claim 1, characterised in that one uses an N-terminal leader part which does not originate from the IgA protease precursor gene.

4. Process according to claim 1, characterised in that one uses the gene coding for the desired protein in a vector which contains the IgA protease precursor gene from micro-organisms of the genus Neisseria in such a manner that the coding gene is positioned within the sequence for the protease and/or the helper segment but not within the sequence for the leader segment of the IgA protease precursor gene.

5. Process according to one of the preceding claims, characterised in that one so introduces the gene coding for the desired protein that the resultant recombinant DNA contains a gene segment coding for an active IgA protease.

6. Process according to claim 5, characterised in that one introduces the coding gene at a position of the IgA protease precursor gene which lies between the natural cleavage positions of the IgA protease precursor protein present in the boundary region of protease and helper segment.

7. Process according to claim 4, characterised in that one introduces the coding gene into the gene segment coding for the IgA protease.

8. Process according to claim 7, characterised in that one additionally introduces a vector which codes the IgA protease into the same host cell or into a further host cell which is cultured simultaneously.

9. Process according to claim 7, characterised in that one brings about the liberation of the protein adhering to the membrane by addition of a specific protease, especially IgA protease, or by chemical agents.

10. Process according to one of the preceding claims, characterised in that, as host cell, there is used a micro-organism of the family Enterobacteriaceae.

11. Use of the plasmid pIP100, contained in E. coli, DSM 3775, as vector for a process according to one of claims 1 to 10.

## Revendications (Revendications pour l'(les) Etat(s) contractant(s) suivant(s): BE, CH, DE, FR, GB, IT, LI, LU, NL, SE)

1. Procédé pour l'obtention par voie génétique et la sécrétion extracellulaire de protéines en utilisant des cellules-hôtes gram-négatives, dans lesquelles on introduit un vecteur recombiné, qui contient au moins le gène codant pour la protéine souhaitée, la transcription de ce gène et la translation,
caractérisé en ce que pour l'obtention extracellulaire de la protéine souhaitée, on met en oeuvre un gène codant pour celle-ci dans un vecteur de telle manière qu'il en résulte un ADN recombiné qui code pour un polypeptide de fusion, contenant :
(i) une fraction de tête N-terminale pour la pénétration des membranes internes des cellules-hôtes gram-négatives,
(ii) une fraction qui contient la protéine souhaitée, et
(iii) une fraction C-terminale à partir du segment-assistant d'un gène-précurseur-protéase-IgA selon la figure 3 pour la pénétration des membranes externes des cellules-hôtes gram-négatives, la fraction à partir du segment-assistant étant sélectionnée de manière à entraîner une sécrétion extracellulaire du polypeptide de fusion.

2. Procédé selon la revendication 1,
caractérisé en ce que l'on utilise une fraction de tête N-terminale à partir du gène-précurseur-protéase-IgA.

3. Procédé selon la revendication 1,
caractérisé en ce que l'on utilise une fraction de tête N-terminale qui ne provient pas du gène-précurseur-protéase-IgA.

4. Procédé selon la revendication 1,
caractérisé en ce que l'on met en oeuvre le gêne codant pour la protéine souhaitée dans un vecteur qui contient le gène-précurseur-protéase-IgA à partir de microorganismes du type Neisseria, de manière à positionner le gène codant à l'intérieur de la séquence pour le segment protéase- ou/et le segment-assistant, mais non à l'intérieur de la séquence pour le segment d'attaque du gène-précurseur-protéase-IgA.

5. Procédé selon l'une des revendications précédentes, caractérisé en ce que l'on insère le gène codant pour la protéine souhaitée de manière que l'ADN recombiné obtenu contienne un segment de gène codant pour une protéase-IgA active.

6. Procédé selon la revendication 5,
caractérisé en ce que l'on insère le gène codant dans une zone du gène-précurseur-protéase-IgA qui se situe entre les points de clivage naturels de la protéine-précurseur-protéase-IgA se trouvant dans la région limitrophe du segment protéase et du segment-assistant.

7. Procédé selon la revendication 4,
caractérisé en ce que l'on introduit le gène codant dans le segment de gène codant pour la protéase-IgA.

8. Procédé selon la revendication 7,
caractérisé en ce que l'on introduit de plus dans la même cellule-hôte ou dans une autre cellule-hôte qui est simultanément cultivée, un vecteur qui code pour la protéase-IgA.

9. Procédé selon la revendication 7,
caractérisé en ce que l'on procède à la libération de la protéine adhérant sur la membrane par addition d'une protéase spécifique, notamment de la protéase-IgA ou par des agents ou moyens chimiques.

10. Procédé selon l'une des revendications précédentes, caractérisé en ce qu'en tant que cellule-hôte, on utilise un microorganisme de la famille des entérobactéries.

11. Utilisation du plasmide pIP100, contenu dans l'E. Coli, DSM 3775 en tant que vecteur pour un procédé selon l'une des revendications 1 à 10.

12. Fragment-ADN-BamHI/EcoRI qui code pour un précurseur-protéase IgA à partir de microorganismes du type Neisseria, selon la figure 3, à partir de pIP 100 (DSM 3775) contenant
a) la séquence de l'acide nucléique codant montrée sur la figure 1,
b) une variation de cette séquence montrée sur la figure 7.

## Revendications (Revendications pour l'(les) Etat(s) contractant(s) suivant(s): GR, AT, ES)

1. Procédé pour l'obtention par génie génétique et la sécrétion extracellulaire de protéines en utilisant des cellules-hôtes gram-négatives, dans lesquelles on introduit un vecteur recombiné, qui contient au moins un gène codant la protéine souhaitée, la transcription de ce gène et la translation,
caractérisé en ce que pour l'obtention extracellulaire de la protéine souhaitée, on met en oeuvre un gène codant pour celle-ci dans un vecteur de telle manière qu'il en résulte un ADN recombiné qui code pour un polypeptide de fusion, contenant :
(i) une fraction de tête N-terminale pour la pénétration des membranes internes des cellules-hôtes gram-négatives,
(ii) une fraction qui contient la protéine souhaitée, et
(iii) une fraction C-terminale à partir du segment-assistant d'un gène-précurseur-protéase-IgA selon la figure 3 pour la pénétration des membranes externes des cellules-hôtes gram-négatives, la fraction à partir du segment-assistant étant sélectionnée de manière à entraîner une sécrétion extracellulaire du polypeptide de fusion.

2. Procédé selon la revendication 1,
caractérisé en ce que l'on utilise une fraction de tête N-terminale à partir du gène-précurseur-protéase-IgA.

3. Procédé selon la revendication 1,
caractérisé en ce que l'on utilise une fraction de tête N-terminale qui ne provient pas du gène-précurseur-protéase-IgA.

4. Procédé selon la revendication 1,
caractérisé en ce que l'on met en oeuvre le gène codant pour la protéine souhaitée dans un vecteur qui contient le gène-précurseur-protéase-IgA à partir de microorganismes du type Neisseria, de manière à positionner le gène codant à l'intérieur de la séquence pour le segment protéase- ou/et le segment-assistant, mais non à l'intérieur de la séquence pour le segment d'attaque du gène-précurseur-protéase-IgA.

5. Procédé selon l'une des revendications précédentes, caractérisé en ce que l'on insère le gène codant pour la protéine souhaitée de manière que l'ADN recombiné obtenu contienne un segment de gène codant pour une protéase-IgA active.

6. Procédé selon la revendication 5,
caractérisé en ce que l'on insère le gène codant dans une zone du gène-précurseur-protéase-IgA qui se situe entre les points de clivage naturels de la protéine-précurseur-protéase-IgA se trouvant dans la région limitrophe du segment protéase et du segment-assistant.

7. Procédé selon la revendication 4,
caractérisé en ce que l'on introduit le gène codant dans le segment de gène codant pour la protéase-IgA.

8. Procédé selon la revendication 7,
caractérisé en ce que l'on introduit de plus dans la même cellule-hôte ou dans une autre cellule-hôte qui est simultanément cultivée, un vecteur qui code pour la protéase-IgA.

9. Procédé selon la revendication 7,
caractérisé en ce que l'on procède à la libération de la protéine adhérant sur la membrane par addition d'une protéase spécifique, notamment de la protéase-IgA ou par des agents ou moyens chimiques.

10. Procédé selon l'une des revendications précédentes, caractérisé en ce qu'en tant que cellule-hôte, on utilise un microorganisme de la famille des entérobactéries.

11. Utilisation du plasmide pIP100, contenu dans l'E. Coli, DSM 3775 en tant que vecteur pour un procédé selon l'une des revendications 1 à 10.
